# EUROPEAN PATENT APPLICATION

(11) **EP 4 644 358 A1**
(43) Date of publication of application: **05.11.2025**
(21) Application number: 24172986.2
(22) Date of filing: 29.04.2024
(51) Int. Cl.: C07C 69/34, C07D 311/76

(54) **PROCESS TO OBTAIN METHYL (E)-2-[2-(HALOMETHYL)-3-METHYL-PHENYL]-3-METHOXY-PROP-2-ENOATES AND INTERMEDIATES THEREOF**

(71) Applicant: BASF SE, 67056 Ludwigshafen am Rhein (DE)
(72) Inventor: BORATE, Kailaskumar, Navi Mumbai, 400705 (IN); KADUSKAR, Rahul, Navi Mumbai, Maharashtra 400705 (IN); RATH, Rakesh, Navi Mumbai, 400705 (IN); THOMBAL, Raju, Navi Mumbai, Maharashtra 400705 (IN); SHYMANSKA, Nataliia, 67056 Ludwigshafen am Rhein (DE); SHINDE, Harish, Navi Mumbai, Mumbai 400705 (IN); GOETZ, Roland, 67056 Ludwigshafen am Rhein (DE)
(74) Representative: BASF IP Association

(57) **Abstract**

The present invention relates to processes to obtain methyl (E)-2-[2-halomethyl)-3-methylphenyl]-3-hydroxy-prop-2-enoates and intermediates thereof. These compounds are used as intermediates for preparing strobilurin type compounds for combating phytopathogenic fungi containing an amino acid substitution F129L in the mitochondrial cytochrome b protein (also referred to as F129L mutation in the mitochondrial cytochrome b gene) conferring resistance to Qo inhibitors.

## Description

The present invention relates to a process for preparation of methyl (*E*)-2-[2-halomethyl)-3-methyl-phenyl]-3-methoxy-prop-2-enoates intermediates for the preparation of substituted enol ether fungicides. The invention also relates to novel intermediates for the preparation of substituted methyl (*E*)-3-methoxy-2-[3-methyl-2-[[(*E*)-1-(het)arylethylideneamino]oxymethyl]-phenyl]prop-2-enoates.

Such substituted methyl (*E*)-3-methoxy-2-[3-methyl-2-[[(*E*)-1-(het)arylethylideneamino]oxymethyl]phenyl]prop-2-enoates are known as fungicides from WO 2021/153754,
WO 2021/219386, WO 2021/219387, WO 2021/219388, WO 2021/219390, WO 2021/249928; WO 2022/008303, WO 2022/013009, WO 2022//03390, WO 2023/072670, WO 2023/072671, WO 2023/072672 and EP 4242198. The preparation of fungicidal compounds is described in the abovementioned references inter alia by a coupling of the intermediates of the formula 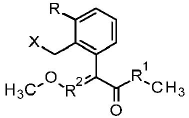 inter alia with substituted benzaldehyde oxime compounds.

It has also been known inter alia from US 2004/0152894 A1 and CN10382722 A1, that methyl (*E*)-3-methoxy-2-(2-chloromethylphenyl)-2-propenoate as a suitable precursor inter alia for picoxystrobin can be prepared from isochromanone, but lacking the desired methyl substitution.

New methods are desirable for preparing methyl (*E*)-2-[2-(halomethyl)-3-methyl-phenyl]-3-methoxy-prop-2-enoates useful as intermediates. Thus, it was an object of the present invention to overcome the disadvantages of the known processes and to provide improved, more economical and production plant friendly processes of such intermediate compounds.

It was found that compounds of formula II can be prepared advantageously from 8-methyl isochroman-3-one compound of formula IV 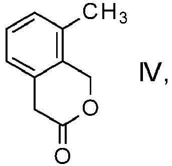 by reaction with a formylating agent to produce compounds of formula III 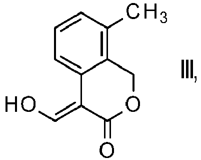 followed by a reaction of compound of formula III
with an alkylating agent to produce a compound of formula II

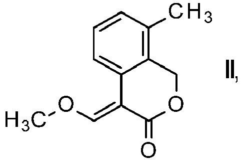

The process according to the present invention differ from process described in US 2004/0152894 A1 and CN 1072667316 by the methyl substitution of the phenyl ring and the use of a base dissolved in methanol thereby avoiding the handling of solid sodium methoxide powder. Further, this process differs by the removal of methanol from the reaction mixture before the etherification step. The dissolved base is easier to handle, in particular in industrial scale production, and less prone to hydrolysis. Surprisingly, it was found that this process according to the invention increases the yield of the desired compound of formula II.

Accordingly, the present invention relates to a process for preparing the compound of formula II 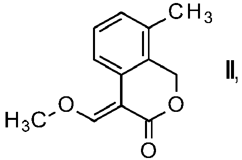 the process comprising:
first, a base selected from alkali metal and alkaline earth metal methoxides dissolved in methanol and an inert solvent are mixed;
second, to this mixture is added the compound of formula IV 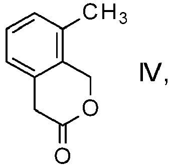
and a formylating agent;
to produce the compound of formula III 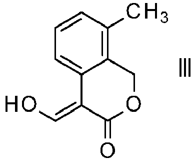
and/or the alkali metal and alkaline earth metal salt thereof,
third, reacting the compound of formula III with an alkylating agent, preferably in the presence of a solvent and a base, optionally in the presence of a catalyst,
wherein
methanol is removed from the mixture
either
before the addition of compound IV and/or the formylating agent, or
after the compound III was produced and before the compound III is reacted with an alkylating agent.

The first formylation reaction requires the presence of a base selected from alkali metal and alkaline earth metal methoxides, such as sodium methoxide and potassium methoxide; even more preferably an alkali metal methoxide is used, in particular sodium methoxide. The methoxide base is dissolved in methanol, preferably sodium methoxide dissolved in methanol which is commercially available. The base dissolved in methanol can be mixed with an inert solvent prior to the reaction. The base is usually provided in excess of compounds IV, preferable 1.1-10 molar equivalents; more preferably 1.1-5 molar eq. of compounds IV; and even more preferably 1.1-3 molar eq..

Suitable inert solvents for the formylation reaction are xylene, benzene, chlorobenzene, cumene, *p*-cymene or toluene, preferably toluene.

Suitable formylating agents are formic acid ester, methyl formate, ethyl formate, propyl formate, isopropyl formate, butyl formate and *t*-butyl formate, preferably methyl formate. The formylating agent is usually provided in excess of compounds IV, preferable 1.1-10 molar equivalents; more preferably 1.1-5 molar eq. of compounds IV; and even more preferably 1.1-3 molar eq..

The formylating agent and the compound IV can be added to the mixture simultaneously or consecutively.

Preferably, the compound IV and/or the formylating agent are dissolved in the abovementioned inert solvent; even more preferably compound IV and the formylating agent are dissolved in such inert solvent.

The formylation reaction may be carried out in a wide temperature range, typically from -10 to 100 °C. A preferred temperature range is from 0 to 25 °C; even more preferably at about temperatures of about 5 to 15 °C.

The compound of formula III produced by the formylation reaction 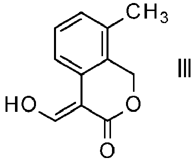 comprises not also its alkali metal and alkaline earth metal salts, preferably alkali metal salts, in particular the sodium salt thereof.

Preferably, the removal of methanol from the mixture is effectuated by distillation e.g. under conditions that allow to retain inert solvent in the mixture, more preferably the removal of methanol via distillation takes place at about 70 °C at ambient pressure or at lower temperatures under reduced pressure, e.g. at about 40 °C under about 350 mbar vacuum. Preferably, this distillation is performed until methanol is completely removed from the mixture. Optionally, the mixture may be cooled thereafter to about 10 °C.

According to one embodiment, the removal of methanol is effectuated before the addition of compound IV and/or the formylating agent.

According to a preferred embodiment, the formylation reaction is completed first before methanol is removed from the mixture as described above. Thus, in this embodiment, methanol is removed after the compound III was produced and before the compound III is reacted with an alkylating agent (etherification reaction), preferably by distillation as described above.

For the second etherification reaction, catalysts can be added. Suitable catalysts are phase transfer catalysts, selected from crown ethers, such as 18-brown-6, azacrown, thiacrown; ionophore; cryptand; phosphonium salts, such as tetrabutyl phosphonium chloride, tetrabutyl phosphonium bromide, benzyltrimethyl phosphonium chloride and benzyltrimethyl phosphonium bromide; or quaternary ammonium salts, such as benzyltrialkylammonium halides and benzyltrialkylammonium hydroxide and tetralkylammonium halides; preferably benzyltrialkylammonium halides, benzyltributylammonium, benzyltrimethylammonium or benzyltriethylammonium halide, particularly preferred as bromide or chloride, in particular benzyltributylammonium chloride; preferably tetraalkylammonium halides, even more preferably selected from Stark's catalyst (Aliquat 336; CAS 5137-55-3; *N*-methyl-*N,N*-dioctyl-1-octanaminiumchloride), tetramethylammonium, tetraethylammonium, tetrapropylammonium and tetra-n-butylammonium halides; wherein the halides are even more preferably selected from bromide and chloride. The catalyst is usually provided at very low amounts usually ranging from 0.01 to 0.5 molar eq. of compounds IV, preferably from 0.01 to 0.2 molar eq., and even more preferably from 0.01 to 0.1 molar eq.. Preferably, the catalyst is added to the reaction mixture prior to compound IV. According to an embodiment, the catalyst is added as an aq. solution.

Preferably, this second etherification reaction is performed in a presence of a base, such as alkali metal and alkaline earth metal hydroxides, preferably LiOH, NaOH, KOH, CsOH, Ba(OH)₂ or Ca(OH)₂, more preferably NaOH or KOH, in particular NaOH. According to one embodiment, the base is added after the catalyst and before the alkylating agent. According to another embodiment, the base is added after the catalyst and after the alkylating agent. According to a further embodiment, the base is added as an aq. solution.

Usually, the etherification reaction is performed in the presence of an inert solvent, preferably in presence of a biphasic mixture of water and water-immiscible solvents such as xylene, benzene, chlorobenzene, cumene, p-cymene or toluene, preferably toluene.

For the etherification reaction, suitable alkylating agents are sulfuric acid esters, such as dimethyl sulfate and diethyl sulfate; or alkyl halides, such as methyl chloride or methyl bromide; in particular dimethyl sulfate. The alkylating agent is usually provided in excess of compounds IV, preferable 1.1-10 molar equivalents; more preferably 2-10 molar eq. of compounds IV; and even more preferably 2-4 molar eq.; and in particular 1.1 to 2 molar eq..

The reaction may be carried out in a wide temperature range, typically from 0 °C to 100 °C. A preferred temperature range is from 10 to 40 °C; even more preferably at ambient temperatures of about 25 °C.

The reaction can be carried out at pressure ranges typically form 0.5 atm to 2 atm, in particular at ambient pressure (0.8 to 1.5 atm).

After completion of the reaction, water may be added to stop the reaction.

After completion of the reaction, the compound of formula III may be obtained by distillation of the organic solvent phase, preferably under reduced pressure.

The invention relates also to compound of formula II

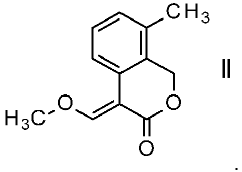

It was also found that compounds of formula I 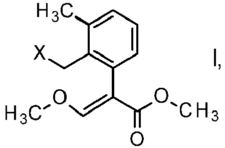 wherein X is Cl or Br;
can be obtained starting from compound of formula II 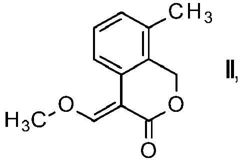 in the absence of an inert solvent.

This process according to the present invention differs from the US 2004/0152894 A1 by the methyl substitution at the phenyl ring and inter alia by the absence of an inert solvent during the halogenation reaction. Surprisingly, it was found that the absence of an inert solvent, in particular an aromatic hydrocarbon solvent, during the halogenation of 8-methyl substituted compound II, reduces undesired side reactions, impurity formation and increases yield of the desired compound of formula I.

Accordingly, the present invention also relates to a process for preparing compounds of formula I 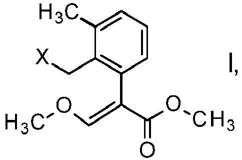 wherein X is Cl or Br;
the process comprising:
first, reacting the compound of formula II 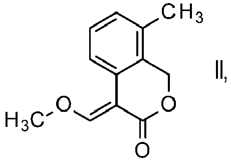
with a halogenating agent in the absence of an inert aromatic hydrocarbon solvent;
optionally in the presence of a catalyst,
second, removing excess of the halogenating agent,
third, reacting the acid halide compound formed with methanol in the presence of a base and preferably of a solvent.

Suitable halogenating agents are thionyl chloride, thionyl bromide, sulfinyl chloride, oxalyl chloride, phosgene, chlorine, bromine, phosphorus trichloride, phosphorus pentachloride, and phosphorous tribromide, preferably thionyl chloride or bromine, in particular thionyl chloride.

The halogenating agent is usually provided in excess of compounds II, preferable 0.9-10 molar equivalents; more preferably 0.9 to 4 molar eq. of compounds II; and even more preferably 2-4 molar eq..

The reaction may be carried out in a wide temperature range, typically from 0 °C to 100 °C. A preferred temperature range is from 10 to 70 °C; more preferably at temperatures of about 25 to 55 °C., even more preferably at temperatures of about 30 to 50 °C.

The reaction can be carried out at pressure ranges typically from 0.5 atm to 2 atm, in particular at ambient pressure (0.8 to 1.5 atm).

Preferably, the halogenation reaction is performed in the absence of an inert solvent, in particular in absence of an aromatic hydrocarbon solvent.

Further, preferably, catalysts can be added to the reaction mixture, more preferably the catalyst is added after the addition of halogenating agent and compound II. Suitable catalysts are *N,N*-dimethylformamide (DMF), *N*-formyl morpholine, *N*-formyl piperidine and pyridine, in particular DMF. The catalyst is usually provided at very low amounts usually ranging from 0.01 to 0.5 molar eq. of compounds II, preferably from 0.01 to 0.2 molar eq., and even more preferably from 0.01 to 0.1 molar eq..

The first part of the reaction is completed by formation of the acyl halide compound of formula Ila 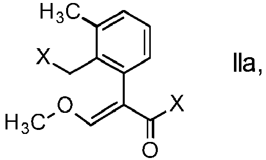 wherein both X are either Cl or Br, preferably both X are Cl.

Preferably, the first part of the reaction is followed by a removal of the excess halogenating agent. Even more preferably, this removal is effectuated by distillation, in particular evaporation under reduced pressure.

Optionally, the halogenating agent, in particular thionyl chloride, may further be removed by addition of an inert solvent, such as, xylene, benzene, chlorobenzene, cumene, p-cymene or toluene, preferably toluene, followed by re-evaporation. This procedure may be repeated more than once.

The second part of the reaction, the esterification of the acyl halide Ila with methanol is usually or preferably carried out in the presence of an inert solvent.

Suitable inert solvents for the esterification are xylene, benzene, chlorobenzene, cumene, p-cymene or toluene, preferably toluene or chlorobenzene, in particular chlorobenzene.

Preferably, the esterification is performed in the presence of a base. Suitable bases are organic bases such as tertiary amines, triethylamine, tri(*n*-butyl)amine, tri(*n*-propyl)amine, diisopropylethylamine, tetramethylenediamine, methylpiperidine, diazabicyclo[3,3,0]octane; and pyridines, piperidines; and inorganic bases such as hydroxide or phosphate salts of Li, K, Na, Ba, Ca and Cs; preferably triethylamine and pyridine, in particular triethylamine.

Methanol is usually provided in excess of compound II, preferable 1.1-30 molar equivalents; more preferably 2-10 molar eq. of compounds IV; and even more preferably 2-4 molar eq..

The reaction may be carried out in a wide temperature range, typically from -10 °C to 100 °C. A preferred temperature range is from -20 to 25 °C; even more preferably at about temperatures of about -10 to 10 °C.

The reaction can be carried out at pressure ranges typically from 0.5 atm to 2 atm, in particular at ambient pressure (0.8 to 1.5 atm).

Optionally, the reaction can be stopped by quenching by addition of water.

Accordingly, the present invention also relates to the novel compound of formula II 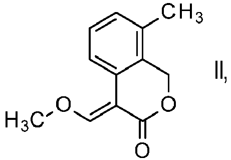 and to the novel compound of formula I 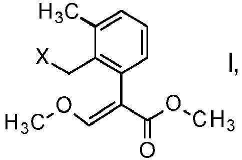 wherein X is Cl, being useful as intermediates for preparing fungicidal methyl (E)-3-methoxy-2-[3-methyl-2-[[(*E*)-1-(het)arylethylideneamino]oxymethyl]phenyl]prop-2-enoates.

### Examples

### Example-1: Synthesis of (4E)-4-(methoxymethylene)-8-methyl-isochroman-3-one (compound II)

### Alternative 1A: Methanol distillation before formylation reaction

Sodium methoxide (30% in methanol, 4.58 ml, 0.025 moles) and toluene (50 ml) were mixed at 25 °C. Methanol (3.10 g) was completely distilled out from reaction mass at 70 °C and reaction mass cooled to 10°C. A solution of 8-methylisochroman-3-one (2.10 g, 0.012 moles) and methyl formate (2.27 ml, 0.036 moles) in toluene (50 ml) was added to the reaction mass for 2 hr at 10°C and further stirred for 2 hr at 10°C. After complete consumption of the starting material, benzyltributylammonium chloride (0.115 g, 0.0004 moles) was added followed by the slow addition of 10% aq. NaOH solution within 20 min (0.7 g, 0.016 moles) and reaction mass warmed to the 25 °C. Then dimethyl sulfate (3.17g, 0.025 moles) was added dropwise over 3 hr at 25 °C and further stirred 6h at same temperature. After completion of the reaction, water (10 ml) was added & toluene layer was separated. The aq. layer was re-extracted with toluene (10 ml). Combined organic layer was washed with brine, dried over sodium sulfate, and evaporated to obtain (4*E*)-4-(methoxymethylene)-8-methyl-isochroman-3-one (2.01 g, Yield: 76%).

### Alternative 1B: - Methanol distillation after first formylation step

Sodium methoxide (30% in methanol, 4.58 ml, 0.025 moles) and toluene (50 ml) were mixed at 25 °C. A solution of 8-methylisochroman-3-one (2.10 g, 0.012 moles) and methyl formate (2.27 ml, 0.036 moles) in toluene (50 ml) was added to the reaction mass for 2 hr at 10 °C and further stirred for 2 hr. Methanol (3.50 g) was completely distilled out from reaction mass at 40 °C under 350 mbar vacuum. Benzyltributylammonium chloride (0.115 g, 0.0004 moles) was added followed by the slow addition of 10% aq. NaOH solution in 20 min (0.7 g, 0.016 moles) at 10 °C, and reaction mass warmed to the 25 °C. Then, dimethyl sulfate (3.17g, 0.025 moles) was added dropwise over 3 hr at 25°C and further stirred 6 hr at the same temperature. After completion of the reaction, water (10 ml) was added and the toluene layer was separated. The aq. layer was re-extracted with toluene (10 ml). Combined organic layer was washed with brine, dried over sodium sulfate, and evaporated to obtain (4*E*)-4-(methoxymethylene)-8-methyl-isochroman-3-one (2.3 g, Yield: 87%).
¹H NMR (500 MHz, DMSO-d₆) δ 7.75 (s, 1H), 7.68 (d, J = 7.9 Hz, 1H), 7.23 (t, J = 7.7 Hz, 1H), 7.07 (d, J = 7.5 Hz, 1H), 5.30 (s, 2H), 4.05 (s, 3H), 2.23 (s, 3H).
¹³C NMR (126 MHz, DMSO) δ 167.67, 161.56, 133.24, 129.10, 128.68, 128.10, 128.02, 124.15, 102.92, 66.78, 63.68, 18.37.

HPLC Method used:

| | |
|---|---|
| Column: | Cortecs 1.6 µm C18+ 30 × 2.1 mm |
| Mobile phase A: | 0.1% TFA in water |
| Mobile phase B: | 0.1% TFA in ACN |
| Flow: | 0.6 ml/min |
| Column oven: | 40°C |
| Gradient: | 5% B in 0 min, hold 5% in 0.02 min, 50% B in 2.5 min, 95% B in 2.9 min, 5% B in 3.2 min, hold 5% B in 4.1 min. Total run time: 4.3 min. |
| Retention times: | comp. IV: 2.48 min; comp. III: 2.74 min; comp. II: 2.95 min |

### Comparative example-2:

### a) Synthesis of (4E)-4-(methoxymethylene)isochroman-3-one (according to CN1072667316)

Solid sodium methoxide (7.29 g, 0.134 moles) and toluene (200ml) were mixed at 28-30 °C followed by dropwise addition of methyl formate (12.49 ml, 0.202 moles) in 30 min and reaction mixture was further stirred for 30 min at the same temperature. Then, 3-isochromone (10 g, 0.067 moles) added to reactor and stirred further for 12 hours. Aqueous solution of tetrabutylammonium bromide (0.652 g, 0.002 moles) was then added at 30 °C followed by dropwise addition of dimethyl sulfate (12.98 ml, 0.134 moles) over 20 min. Aqueous solution of potassium carbonate (3.49 g, 0.0873 moles) and further reaction was stirred at same temperature for one hour. Upon completion the reaction was added water (50 ml) was added and the toluene layer was separated. The aq. layer was re-extracted with toluene (50 ml). The combined organic layer was washed with brine, dried over sodium sulfate, and evaporated to obtain (4*E*)-4-(methoxymethylene)isochroman-3-one (10.56 g, Yield: 82.33%).

### b) Synthesis of (4E)-4-(methoxymethylene)isochroman-3-one (according to the process of the present invention)

Sodium methoxide (30 % in methanol, 24.12 ml, 0.134 moles) and toluene (100 ml) were mixed at 25°C. A solution of 3-isochromone (10 g, 0.067 moles) and methyl formate (12.49 ml, 0.202 moles) in toluene (100 ml) was added to the reaction mass for 2 hr at 10 °C and further stirred for 2 hr. Methanol (19.09 g) was completely distilled out from reaction mass at 40 °C under 350 mbar vacuum. Benzyltributylammonium chloride (0.625 g, 0.0002 moles) was added followed by the slow addition of 10% aq. NaOH solution in 20 min (3.49 g, 0.0873 moles) at 10°C, and reaction mass warmed to the 25°C. Then, dimethyl sulfate (15.45g, 0.134 moles) was added dropwise over 3 hr at 25°C and further stirred 6 hr at the same temperature. After completion of the reaction, water (50 ml) was added and the toluene layer was separated. The aq. layer was re-extracted with toluene (50 ml). Combined organic layer was washed with brine, dried over sodium sulfate, and evaporated to obtain (4*E*)-4-(methoxymethylene)isochroman-3-one (11.63 g, Yield: 90.65%).

This comparative example 2 shows that the use of sodium methoxide dissolved in methanol and the removal of methanol before the etherification reaction improves the yield of the desired product in comparison to the procedure of the prior art.

### Comparative Example 3: Synthesis of methyl (E)-2-[2-(chloromethyl)-3-methyl-phenyl]-3-methoxy-prop-2-enoate (compound I, wherein X is Cl) in presence of an aromatic hydrocarbon solvent analogous to US 2004/0152894 A1

Thionyl chloride (1.74 g, 0.0146 moles) was added dropwise to the mixture of (4*E*)-4-(methoxymethylene)-8-methyl-isochroman-3-one (product of example 3; 1 g, 0.0048 moles), DMF (0.071 g, 0.0009 moles) in chlorobenzene (5 ml) at 25°C and stirred for 16 hr at 75 °C. Excess thionyl chloride was completely distilled out from the reaction mass and was added 2ml of MCB. This reaction mass was slowly added over 15 min to the solution of methanol (1.5 g, 0.048 moles), triethyl amine (0.49 g, 0.0048, moles) in chlorobenzene (2 ml) at 0 °C. Then the reaction mass was further stirred for 30 min at same temperature. After completion of the reaction, the reaction was quenched with water (10 ml) and chlorobenzene layer was separated. The aq. layer was re-extracted with chlorobenzene (5 ml). The combined organic layer was washed with brine and evaporated to obtain methyl (*E*)-2-[2-(chloromethyl)-3-methyl-phenyl]-3-methoxy-prop-2-enoate (0.74 g, Yield: 60%).

### Example 4: Synthesis of methyl (E)-2-[2-(chloromethyl)-3-methyl-phenyl]-3-methoxy-prop-2-enoate (compound I, wherein X is Cl) in absence of an inert solvent

(4*E*)-4-(methoxymethylene)-8-methyl-isochroman-3-one (1 g, 0.0048 moles) was added to reaction flask containing thionyl chloride (3.4 g, 0.0293 moles) portion wise at about 25 °C. Then, DMF (0.071 g, 0.0009 moles) was added at 25 °C and stirred for 16 hr at 50 °C. After reaction completion, excess of thionyl chloride was completely distilled out under reduced pressure followed by two stripping of toluene to remove traces of thionyl chloride. This reaction mass was dissolved in 5 ml toluene and was dosed to reaction flask containing methanol (1.5 g, 0.048 moles) and triethylamine (0.49 g, 0.0048 moles) maintained at 0 °C over 30 mins. Then the reaction mass was quenched by addition of water at 10 °C. The toluene layer was separated and washed with brine, on evaporation of this layer obtained methyl (*E*)-2-[2-(chloromethyl)-3-methyl-phenyl]-3-methoxy-prop-2-enoate (0.96g, Yield: 77%).
¹H NMR (300 MHz, DMSO-d₆): δ 7.69 (s, 1H), 7.29 - 7.13 (m, 2H), 6.91 (d, J = 6.2 Hz, 1H), 4.56 (s, 2H), 3.79 (s, 3H), 3.59 (s, 3H), 2.41 (s, 3H).
¹³C NMR (75 MHz, DMSO): δ 167.54, 161.46, 138.14, 134.68, 134.37, 130.32, 129.45, 128.80, 109.50, 62.13, 51.67, 42.41, 19.37.

HPLC Method used:

| | |
|---|---|
| Column: | Phenomenex 2.6 µm Kinetex C18 150 × 4.6 mm |
| Mobile phase A: | 0.1% TFA in water |
| Mobile Phase B: | 0.1% TFA in acetonitrile (ACN) |
| Flow: | 1.2 ml/min |
| Column oven: | 50°C |
| Gradient: | 30% B in 0 min, 40% B in 5 min, 65% B in 8 min, 75% B in 11.5 min, 95% B in 12 min, 30% B in 12.01 min; total run time: 14min. |
| Retention times: | comp. II: 3.26 min; comp I, wherein X is Cl: 5.01 min |

## Claims

1. A process for preparing the compound of formula II 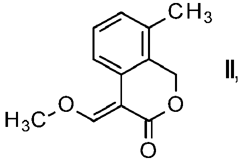 the process comprising:
first, a base selected from alkali metal and alkaline earth metal methoxides dissolved in methanol and an inert solvent are mixed;
second, to this mixture is added the compound of formula IV 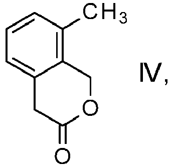
and a formylating agent;
to produce the compound of formula III 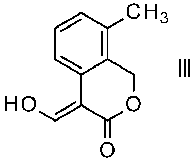
and/or the alkali metal and alkaline earth metal salt thereof,
third, reacting the compound of formula III with an alkylating agent,
wherein
methanol is removed from the mixture
either
before the addition of compound IV and/or the formylating agent, or
after the compound III was produced and before the compound III is reacted with an alkylating agent.

2. The process according to claim 1, wherein the base in the first step is toluene.

3. The process according to claim 1 or 2, wherein the inert solvent in the first step is sodium methoxide.

4. The process according to any of the claims 1 to 3, wherein the formylating agent is methyl formate.

5. The process according to any of the claims 1 to 4, wherein the alkylating agent is dimethyl sulfate.

6. The process according to any of the claims 1 to 5, wherein methanol is removed after the compound III is produced and before compound III is reacted with an alkylating agent.

7. The process according to any of the claims 1 to 6, wherein the reaction of compound of formula III with an alkylating agent is effectuated in the presence of an inert solvent and a base.

8. The process according to claim 7, wherein the inert solvent is a biphasic mixture of water and toluene.

9. The process according to claim 7 or claim 8, wherein the base for the reaction of the compound of formula III with an alkylating agent is selected from NaOH and KOH.

10. The process according to any of the claims 1 to 9, wherein the reaction of compound of formula III with an alkylating agent is effectuated in the presence of a catalyst.

11. The process according to claim 10, wherein the catalyst is selected from quaternary ammonium salts.

12. The process according to claim 11, wherein the quaternary ammonium salt is selected from benzyltrialkylammonium and tetraalkylammonium halides.

13. A process for preparing the compound of formula I 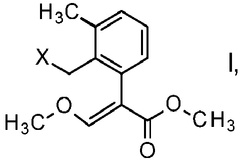 wherein X is Cl or Br;
the process comprising:
first, reacting the compound of formula II 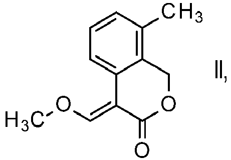
with a halogenating agent in the in the presence of a catalyst and in absence of an inert solvent;
second, removing excess of the halogenating agent,
third, reacting the acid halide compound formed with methanol in the presence of a base.

14. The process according to claim 13, wherein the halogenating agent is thionyl chloride.

15. The process according to claim 13 or 14, wherein the catalyst is *N,N*-dimethylformamide.

16. The process according to any of the claims 13 to 15, wherein the base is selected from triethylamine and pyridine.

17. The process according to any of the claims 13 to 16, wherein the reaction of the compound of formula II with a halogenating agent is carried out at temperatures of from 25 to 55 °C..

18. The process according to any of the claims 13 to 17, wherein the reaction of the acid halide compound with methanol takes place in the presence of an inert solvent selected from toluene and chlorobenzene.

19. The process according to any of the claims 13 to 18, wherein the compound of formula II has been obtained according to the process as defined in any of the claims 1 to 11.

20. The compound of formula II

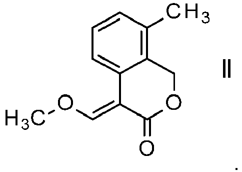

21. The compound of formula I 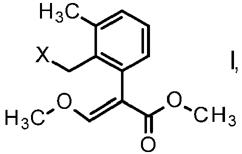 wherein X is Cl.
